# EUROPEAN PATENT APPLICATION

(11) **EP 4 296 266 A1**
(43) Date of publication of application: **27.12.2023**
(21) Application number: 22755576.0
(22) Date of filing: 18.02.2022
(51) Int. Cl.: C07D 237/14, C07D 401/04, C07D 403/04, A61K 31/50, A61K 31/501, A61P 3/10, A61P 25/00, A61P 25/28, A61P 29/00, A61P 33/06, A61P 35/00

(54) **S-CONFIGURATION-CONTAINING AMINO BENZAMIDE PYRIDAZINONE COMPOUND, PREPARATION METHOD THEREFOR, AND PHARMACEUTICAL COMPOSITION AND APPLICATION THEREOF**

(30) Priority: 20.02.2021 CN 202110193453
(71) Applicant: Shanghai Institute of Materia Medica, Chinese Academy of Sciences, Pudong, Shanghai 201203 (CN)
(72) Inventor: HU, Youhong, Shanghai 201203 (CN); GENG, Meiyu, Shanghai 201203 (CN); LI, Daqiang, Shanghai 201203 (CN); SHEN, Aijun, Shanghai 201203 (CN); ZHANG, Zhuo, Shanghai 201203 (CN); LI, Yalei, Shanghai 201203 (CN); YANG, Huajie, Shanghai 201203 (CN); LIU, Hongchun, Shanghai 201203 (CN); ZHONG, Hanyue, Shanghai 201203 (CN); DING, Jian, Shanghai 201203 (CN)
(74) Representative: Petraz, Gilberto Luigi
(86) International application number: PCT/CN2022/076727
(87) International publication number: WO 2022/174803

(57) **Abstract**

The present invention relates to an S-configuration-containing amino benzamide pyridazinone compound, a preparation method therefor, and a pharmaceutical composition and application thereof. Specifically, the present invention relates to a compound represented by the following general formula I or a pharmaceutically acceptable salt thereof, a preparation method therefor, and a pharmaceutical composition and application thereof. The S-configuration compound of the present application has very strong binding activity on class I histone deacetylase (HDAC1), and shows inhibitory activity on in-vitro proliferation of various tumor cells.

## Description

### Technical field

The present invention relates to a series of amino benzamide pyridazinone compounds, more specifically, the present invention relates to a series of S-configuration-containing amino benzamide pyridazinone compounds, their preparation method, pharmaceutical composition, and use in preparation of a medicament for preventing or treating a disease associated with the abnormal activity and expression of class I histone deacetylase (class I HDAC).

### Background

The acetylation levels of histones or non-histone proteins within cells are regulated by histone acetyltransferase (HAT) and histone deacetylase (HDAC), and can affect cell cycle, differentiation, angiogenesis, and apoptosis, etc. Overexpression of HDAC promotes deacetylation of histone or non-histone, leading to chromatin condensation, and thus inhibiting the transcription of tumor suppressor genes. Research results show that epigenetic functions can be remodeled by inhibiting the activity of HDAC, which has been proved to be an effective method to treat malignant tumors (Nat. Rev. Drug. Discov. 2014, 13 (9), 673-691).

So far, FDA has approved four HDAC inhibitors for the treatment of skin T-cell lymphoma (CTCL), peripheral T-cell lymphoma (PTCL) and multiple myeloma (MM), including the first HDAC inhibitor Vorinostat (SAHA) approved in 2006, Romidepsin (FK228) approved in 2009, Belinastat (PXD-101) approved in 2014, and Panobinostat (LBH589) approved in 2015. Another class of amino benzamide compounds belongs to class I HDAC inhibitors, among which the most representative compounds include Chidamide (CS055), which was approved by NMPA for the treatment of PTCL, and for the treatment of HR+ breast cancer in combination with Exemestane, but its efficacy is insufficient when used alone; and Entinostat (MS-275), now in clinical phase III, which is highly toxic with a low therapeutic index, limiting its further clinical application (Adv. Cancer. Res. 2018, 138, 183-211). In addition, there are a large number of HDAC inhibitors at different stages of drug development.

CN109280032A disclosed a series of compounds sharing a pyridazinone backbone that could be used as HDAC inhibitors for treatment of a mammalian disease associated with abnormal activity and expression of HDAC, such as tumors, inflammation, etc., as exemplified by the representative compounds HYH-072 and HYH-073, which exhibited good inhibitory activities on enzyme and tumor cell proliferation, but these representative compounds are racemates.

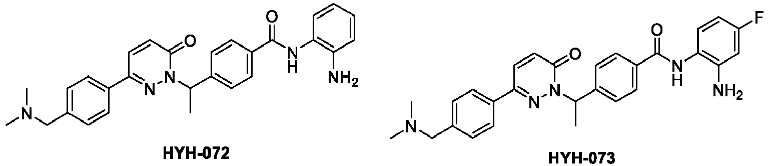

### Summary of the invention

In further research, the inventors have found that amino benzamide pyridazinone compounds with different configurations show significantly different inhibitory activities on enzymes and cell proliferation, and the S-configuration-containing amino benzamide pyridazinone compounds have higher activities, and thus completed the present invention.

The first object of the present invention is to provide a series of S-configuration-containing amino benzamide pyridazinone compounds, or pharmaceutically acceptable salts thereof.

The second object of the present invention is to provide a method for preparing these compounds.

The third object of the present invention is to provide a pharmaceutical composition comprising a therapeutically effective amount of one or more selected from the group consisting of the aforementioned compounds and pharmaceutically acceptable salts thereof.

The fourth object of the present invention is to provide use of the aforementioned compounds or pharmaceutically acceptable salts or the aforementioned pharmaceutical composition in preparation of a medicament for prevention or treatment of a disease associated with abnormal activity and expression of class I histone deacetylase (class I HDAC).

In one aspect, the present invention provides a compound represented by formula I or a pharmaceutically acceptable salt thereof, 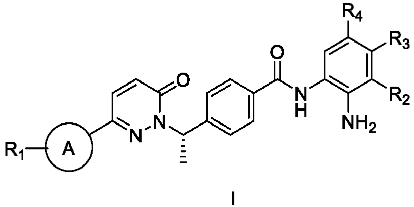 wherein
ring A is selected from a C₆-C₁₀ aryl group or a 5-10 membered heteroaryl group containing 1-3 heteroatoms selected from N, O, and S;
R₁ represents one or more substituents, each independently selected from H, halogen, C₁-C₆ linear or branched alkyl, C₁-C₆ linear or branched alkoxy, and (Rₐ)(R_{b})N(CH₂)-;
wherein Rₐ and R_{b} are each independently a C₁-C₃ alkyl, or form a 3-7-membered heterocyclyl together with the N atom connected therewith;
R₂, R₃, and R₄ are same or different from each other and are independently selected from hydrogen, halogen, C₁-C₃ alkyl, and C₁-C₃ alkoxy.

In some embodiments, ring A is selected from phenyl, pyridyl, and pyrazolyl.

In some embodiments, R₁ is selected from 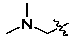 , halogen, C₁-C₃ alkoxy, and C₁-C₃ alkyl.

In some embodiments, the compound is selected from: 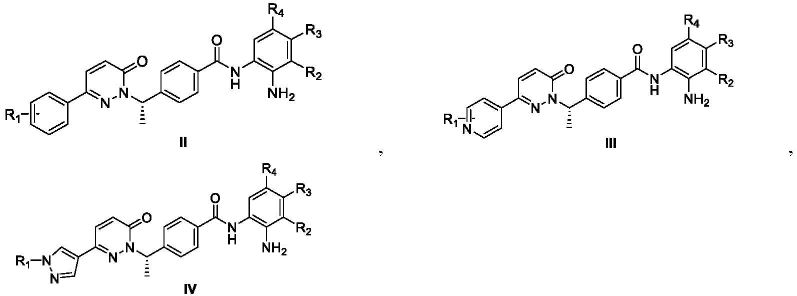 and wherein, R₁, R₂, R₃, and R₄ are each defined as above.

In some embodiments, the compound is selected from the following compounds:

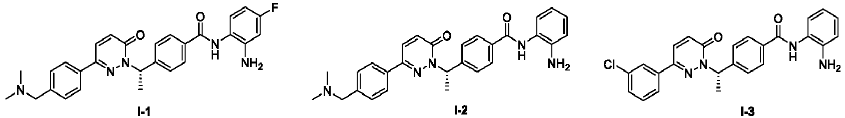

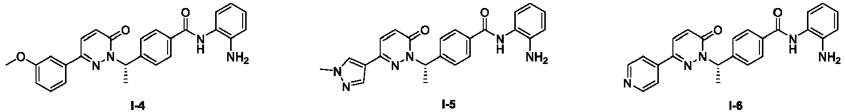

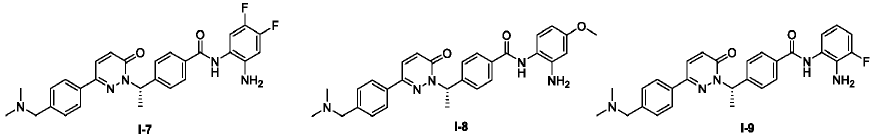

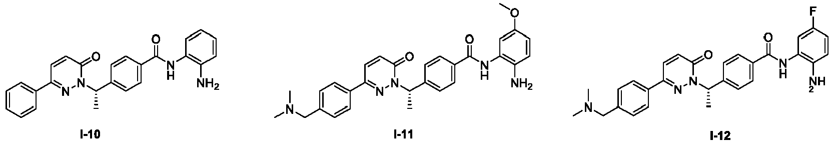

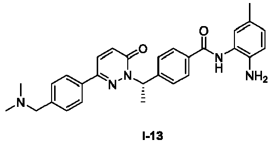

The compound of the present invention may exist in the form of a solvate (including a hydrate), a polymorph, an isotopic labeled compound (such as a deuterate), a tautomer or a prodrug, and these existing forms are also included in the protection scope of the present invention.

In the second aspect, the present invention provides a method for preparing the above compound, which is one of the following schemes,

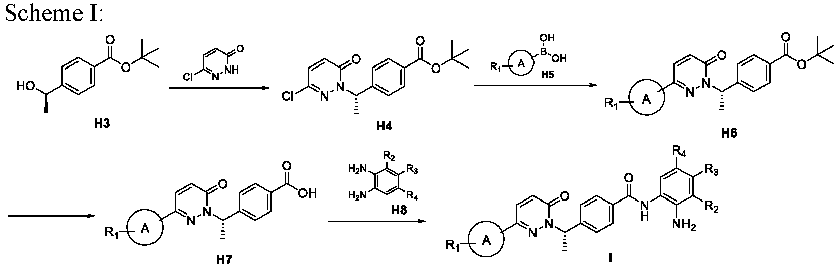

The method comprises steps of:
(1) reacting tert-butyl (R)-4-(1-hydroxyethyl)benzoate of **H3** with 6-chloropyridazinone via Mitsunobu reaction to obtain tert-butyl (*S*)-4-(1-(3-chloro-6-oxo-pyridazin-1(6*H*)-yl)ethyl)benzoate of **H4** with reverse configuration;
(2) reacting tert-butyl (*S*)-4-(1-(3-chloro-6-oxo-pyridazin-1(6*H*)-yl)ethyl)benzoate of **H4** with the compound of **H5** via Suzuki coupling reaction to obtain the compound of **H6**;
(3) removing the tert-butyl protection group of the compound of **H6** to obtain the compound of **H7**;
(4) reacting the compound of **H7** with the compound of **H8** via condensation reaction to obtain the compound of **formula I**;

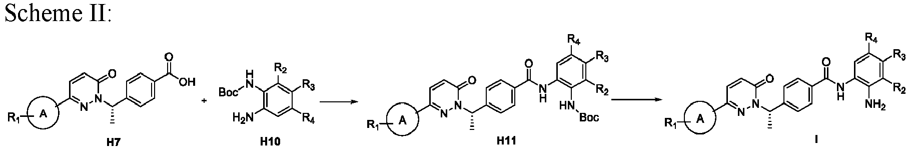

The method comprises steps of:
(1) reacting the compound of **H7** with the compound of **H10** via condensation reaction to obtain the compound of **H11**;
(2) removing the Boc protection group of the compound of **H11** to obtain the compound of **formula I**;

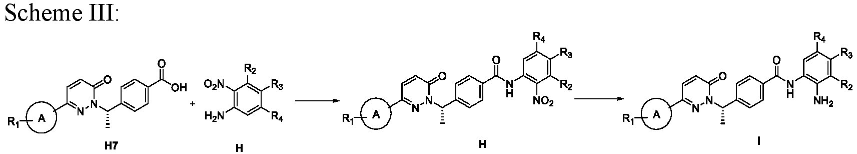

The method comprises steps of:
(1) reacting the compound of **H7** with the compound of **H13** via condensation reaction to obtain the compound of **H14**;
(2) reducing the compound of **H14** to obtain the compound of **formula I**;
wherein, ring A, R₁, R₂, R₃, and R₄ are defined as above.

In the method, the chiral intermediate **H3** can be commercially available or self-synthesized.

In one embodiment, **H3** can be synthesized as follows: 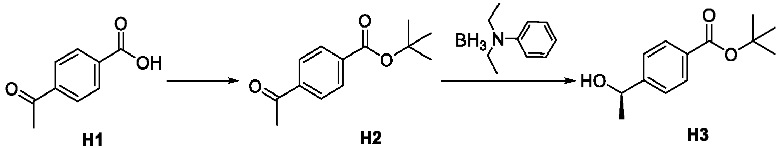
(1) p-acetylbenzoic acid of **H1** is esterified with Boc anhydride to obtain tert-butyl p-acetylbenzoate of **H2**;
(2) tert-butyl (R)-4-(1-hydroxyethyl)benzoate of **H3** is obtained from the reduction reaction of tert-butyl p-acetylbenzoate of **H2** with *N,N-*diethylaniline borane complex in the presence of (*S*)-2-methyl-CBS-oxazole borane ((*S*)-2-Me-CBS) as a chiral catalyst.

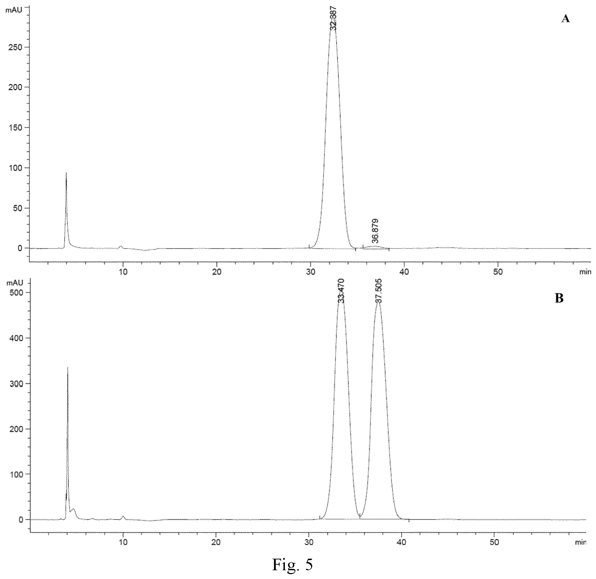

The synthesis process of the above chiral intermediate H3 is simple, and has a high chiral ee value (97.2%, see Fig. 5), and thus it is suitable for industrial production.

In a third aspect, the present invention provides an intermediate of **H7**: 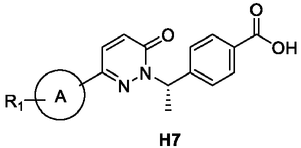 wherein, ring A and R₁ are as defined above.

In a fourth aspect, the present invention provides a pharmaceutical composition comprising at least one selected from the above compounds and pharmaceutically acceptable salts thereof. The pharmaceutical composition may also comprise a pharmaceutically acceptable carrier.

In a fifth aspect, the present invention provides use of the above compound or a pharmaceutically acceptable salt thereof, or the above pharmaceutical composition in preparation of a medicament for prevention or treatment of a disease associated with abnormal activity and expression of class I histone deacetylase (class I HDAC).

In some embodiments, the disease associated with the abnormal activity and expression of class I histone deacetylase (class I HDAC) includes cancer, inflammation, neurodegenerative diseases, malaria or diabetes.

In some embodiments, the cancer is selected from the group consisting of myelodysplastic syndrome, leukemia (including monocyte leukemia, acute myeloid leukemia, Down's syndrome acute megakaryocyte leukemia, T-cell acute lymphoblastic leukemia, acute lymphoblastic leukemia, multiple myeloid leukemia, chronic myeloid leukemia, human T-lymphocyte leukemia, and acute myeloid leukemia), lymphomas (including diffuse large B-cell lymphoma, mantle cell lymphoma, primary cutaneous T-cell non Hodgkin lymphoma, primary mediastinal large B-cell lymphoma, Hodgkin's lymphoma), multiple myeloma, lung cancer, kidney cancer, gastric cancer, breast cancer, melanoma, colon cancer, liver cancer, ovarian cancer, pancreatic cancer, and rectal cancer.

It should be understood that within the scope of the present invention, the above-mentioned technical features and the specific technical features described below (such as the examples) can be combined with each other to form new or preferred technical solutions. They are not elaborated herein due to space constraints.

### Beneficial effects

After studying the structure-activity relationship of this class I HDAC inhibitor of S-configuration-containing amino benzamide pyridazinone compounds, the present inventors have found that for those compounds of which the benzyl connected at 2 position of the pyridazinone has an S configuration, their inhibitory activities on HDAC1, HDAC2 and HDAC3 subtypes, and on the proliferation of human myelodysplastic syndrome cell SKM-1 and human colon cancer cell HCT-116 are significantly superior to those compounds having R configuration, and also higher than those of Chidamide and MS-275, indicating that the S-configuration compounds play a major role in molecular and cellular activity.

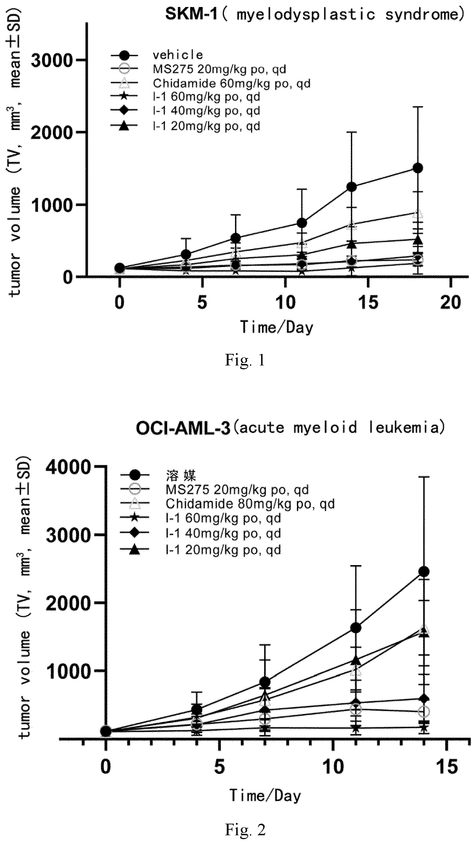

More importantly, the inhibitory effects of the compound provided by the present invention on human myelodysplastic syndrome cell SKM-1 (see Fig. 1) and acute myeloid leukemia cell OCI-AML-3 (see Fig. 2) mice xenograft tumor models are much better than the marketed drug Chidamide at the same dose, and its safety is better than MS-275 under the equivalent efficacy. After continuous oral administration of the compound 1-1 at 60 mg/kg for 18 days, it had therapeutic effect on human myelodysplastic syndrome cells SKM-1 NOD/SCID mice transplanted tumor, and could effectively inhibit the proliferation of mice transplanted tumor with a relative tumor proliferation rate (T/C) of 11.78%, which was significantly superior to the efficacy of the experimental group in which Chidamide was orally administered at 60 mg/kg qd (T/C was 73.45%), and superior to the efficacy of the experimental group in which MS-275 was orally administered at 20 mg/kg qd (T/C was 18.23%). After continuous oral administration of the compound 1-1 at 60 mg/kg for 14 days, it had therapeutic effect on human acute myeloid leukemia cells OCI-AML-3 NOD/SCID mice transplanted tumor, and could effectively inhibit the proliferation of mice transplanted tumor with a relative tumor proliferation rate (T/C) of 7.75%, which was significantly superior to the efficacy of the experimental group in which Chidamide was orally administered at 80 mg/kg qd (T/C was 69.91%), and superior to the efficacy of the experimental group in which MS-275 was orally administered at 20 mg/kg qd (T/C was 14.16%). Besides, during the experiment, the body weight of mice remained basically unchanged, indicating that the compound 1-1 had good safety.

In summary, the compounds provided by the present invention have significantly improved activity, good oral absorption effect, and potent efficacy, thus having better development prospects.

In addition, the compounds of the present invention also have excellent inhibitory activity against a variety of solid tumors (such as lung cancer, kidney cancer, stomach cancer, breast cancer, melanoma, colon cancer, liver cancer, lymphoma, etc.).

### Brief Description of Drawings

Fig. 1 shows the pharmacodynamic results of the compound I-1 of the present application on human myelodysplastic syndrome SKM-1 mice transplanted tumor model.
Fig. 2 shows the pharmacodynamic results of the compound I-1 of the present application on human acute myeloid leukemia OCI-AML-3 mice transplanted tumor model.
Fig. 3 shows the pharmacodynamic results of the compounds I-1 and I-2 of the present application on human malignant melanoma A375 mice transplanted tumor model.
Fig. 4 shows the ee values of the compound I-1 of the present application before enantioselective synthesis (B) and after enantioselective synthesis (A).
Fig. 5 shows the ee value of the intermediate compound H in Example 1 of the present application before enantioselective synthesis (B) and after enantioselective synthesis (A).

### Detailed Description of the invention

### Terms

In the present invention, unless otherwise specified, the meanings of substituents are defined as follows.

The C₆-C₁₀ aryl group refers to an aromatic carbocyclic group with 6 to 10 carbon atoms on the ring, and its specific examples include phenyl, naphthyl, etc.

The halogen atom refers to F, Cl, Br, or I.

The C₁-C₆linear or branched alkyl refers to a linear or branched alkyl with 1 to 6 carbon atoms, and its specific examples include methyl, ethyl, n-propyl, isopropyl, n-butyl, isobutyl, tert-butyl, sec-butyl, n-pentyl, 1-methylbutyl, 2-methylbutyl, 3-methylbutyl, isopentyl, 1-ethylpropyl, neopentyl, n-hexyl, 1-methylpentyl, 2-methylpentyl, 3-methylpentyl, isohexyl, 1,1-dimethylbutyl, 2,2-dimethylbutyl, 3,3-dimethylbutyl, 1,2-dimethylbutyl, 1,3-dimethylbutyl, 2,3-dimethylbutyl, 2-ethylbutyl, etc.

The C₁-C₆ linear or branched alkoxy refers to a linear or branched alkoxy with 1 to 6 carbon atoms, and its specific examples include methoxy group, ethoxy, n-propoxy, isopropoxy, n-butoxy, isobutyloxy, tert-butoxy, sec-butoxy, n-pentoxy, isopentoxy, neopentoxy, n-hexoxy, isohexoxy, 3-methylpentoxy, etc.

The 5-10 membered heteroaryl group containing 1-3 heteroatoms selected from N, O and S refers to an aromatic ring with 5 to 10 atoms on the ring and containing 1-3 heteroatoms selected from N, O and S, which can be monocyclic or bicyclic, such as pyridine ring, pyrrole ring, pyrimidine ring, pyrazine ring, pyridazine ring, thiophene ring, furan ring, pyrazole ring, imidazole ring, oxazole ring, thiazole ring, indole ring, azaindole ring, naphthyridine ring, benzimidazole ring, pyridinoimidazole ring, pyrimidinoimidazole ring, or quinoline ring, etc.

The pharmaceutically acceptable salt, for example, can be a mineral acid salt, such as hydrochloride, hydrobromide, hydroiodate, sulfate, nitrate, phosphate, carbonate, etc; an organic acid salt, such as formate, acetate, propionate, oxalate, malonic acid, succinate, fumarate, maleate, adipate, lactate, malate, citrate, citrate, tartrate, carbonate, picrate, methanesulfonate, ethanesulfonate, p-toluene sulfonate, glutamate, dihydroxynaphthalate, etc.

The pharmaceutically acceptable salt of the S-configuration-containing amino benzamide pyridazinone compound of formula I provided by the present invention can be prepared by dissolving the S-configuration-containing amino benzamide pyridazinone compound of formula I in an alcohol solution or dioxane solution saturated with the corresponding acid for reaction, for example, dissolving the S-configuration-containing amino benzamide pyridazinone compound provided by the present invention in a HCl saturated dioxane solution, stirring at room temperature for 30 minutes, and evaporating to remove the solvent, to prepare the corresponding hydrochloride.

The compound of the present invention may exist in its prodrug, and therefore, the prodrug is also included in the protection scope of the compound of the present invention. The prodrug refers to a compound obtained by chemical structural modification of a drug, which is inactive or less active *in vitro,* and releases an active drug through enzymatic or non-enzymatic transformation in vivo to exert its efficacy.

The form of the prodrug in the present invention is not specially limited, as long as it releases an active drug through enzymatic or chemical action in vivo, so as to exert expected pharmacological effects, and it can be a carrier prodrug or a biological precursor.

The compound of the present invention may exist in non-solvation form and solvation form (also called solvate) containing an pharmaceutically acceptable solvent (such as water, ethanol, etc.). The compounds of the present invention include the solvation and non-solvation forms. The solvate is a complex formed by the compound of formula I and a pharmaceutically acceptable solvent. Optionally, the pharmaceutically acceptable solvent includes water, ethanol, acetic acid, N,N-dimethylformamide or dimethyl sulfoxide, etc.

The compound of the present invention may also exist in different tautomeric forms, all of which are included in the scope of the present invention. The term "tautomer" or "tautomeric form" refers to structural isomers of different energies that are transformed into each other via a low energy barrier.

The present invention also covers the compound of the present invention labeled with an isotope, which is the same as that described herein, except that one or more atoms thereof are replaced by atoms whose atomic mass or mass number is different from that of the atom common in nature. Examples of isotopes that can be included in the compounds of the present invention include isotopes of hydrogen, carbon, nitrogen, oxygen, phosphorus, sulfur, fluorine, iodine, and chlorine, such as ²hydrogen, ³hydrogen, ¹¹carbon, ¹³carbon, ¹⁴carbon, ¹³nitrogen, ¹⁵nitrogen, ¹⁵oxygen, ¹⁷oxygen, ¹⁸oxygen, ³¹phosphorus, ³²phosphorus, ³⁵sulfur, ¹⁸fluorine, ¹²³iodine, ¹²⁵iodine, and ³⁶chlorine, respectively. The isotope labeled compounds of the present invention can generally be prepared by replacing non isotope labeled reagents with isotope labeled reagents, following a method similar to that disclosed in the scheme and/or the following examples.

The pharmaceutically acceptable carrier refers to a conventional drug carrier in the pharmaceutical field, for example, a diluent such as water, etc.; a filler, such as starch, sucrose, etc; a binder, such as a cellulose derivative, alginate, gelatin, polyvinylpyrrolidone; a wetting agent, such as glycerol; a disintegrator, such as agar, calcium carbonate, and sodium bicarbonate; an absorption enhancer, such as a quaternary ammonium compound; a surfactant, such as hexadecane alcohol; an adsorption carrier, such as kaolin and soapy clay; a lubricant, such as talc, calcium stearate and magnesium stearate, and polyethylene glycol, etc. In addition, other auxiliary agent, such as a fragrance and a sweetener, etc., can also be added to the aforementioned pharmaceutical composition.

The present invention will be further illustrated with reference to specific examples. It should be understood that these examples are only used to illustrate the present invention and not to limit the scope of the present invention.

The preparation process of the S-configuration-containing amino benzamide pyridazinone compound provided by the present invention and its biological activities as a Class I HDAC inhibitor will be described in details by way of the following examples, but the present invention is not limited to these examples.

In the following examples, the proton nuclear magnetic resonance was recorded on BrukerAMX-400, BrukerAMX-500 or AMX-600 nuclear magnetic resonance instruments, and the unit of the chemical shift δ was ppm. Unless otherwise specified, all reaction solvents were purified using conventional methods. The silica gel (200-300 mesh) used for column chromatography was produced by Qingdao Ocean Chemical Branch. Unless otherwise specified, all solvents were analytical pure reagents, and the reagents used were purchased from Sinopharm Chemical Reagents Co., Ltd. Ultraviolet fluorescence method was used for color development. The organic solvents were evaporated under reduced pressure in a rotary evaporator.

### Example 1: Synthesis of compound I-1

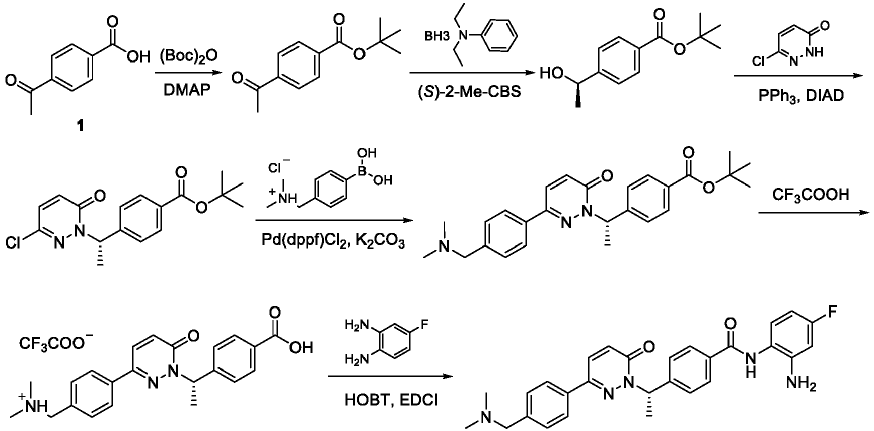

p-Acetyl benzoic acid (1, 100.0 g, 609 mmol) was placed in a 2 L three neck flask, and 300 ml of tetrahydrofuran was added thereto and cooled in an ice bath. 4-dimethylaminopyridine (DMAP, 14.88 g, 121 mmol) was added thereto, the internal temperature of the reaction system was maintained at 10 °C, and a solution of (Boc)₂O (279.1 g, 1278.9 mmol) in 700 ml of tetrahydrofuran (THF) was added dropwise. After the dropwise addition, the reaction was carried out at room temperature for 24 hours. After the reaction was complete as monitored by TLC, the solvent was removed under reduced pressure, 750 ml of ethyl acetate was added, followed by washing with 0.1N hydrochloric acid (2 × 750 ml), saturated sodium bicarbonate solution (2 × 750 ml), and saturated saline (2 × 500 ml) in sequence. After the organic phase was dried with anhydrous sodium sulfate, and removed off the solvent under reduced pressure, 750 ml of n-heptane was added. The resultant was filtered with silica gel to obtain 130 g of a light yellow solid (compound 2), with a yield of 97%. ¹H NMR (400 MHz, CDCl₃) δ 8.06 (d, *J =* 8.7 Hz, 2H), 7.98 (d, *J =* 8.7 Hz, 2H), 2.64 (s, 3H), 1.61 (s, 9H); ESI-MS: m/z = 221[M+H]⁺.

(*S*)-2-methyl-CBS-oxazaborolidine ((*S*)-2-Me-CBS, 8.2 g, 29 mmol), *N*,*N*-diethylaniline borane complex (96.2 g, 590 mmol) and 300 ml of anhydrous methyl tert-butyl ether were added into a 2L three necked flask under the protection of nitrogen, cooled in an ice bath to 10 °C, and added dropwise with a solution of tert-butyl 4-acetylbenzoate (2, 130 g, 590 mmol) in 300 ml of anhydrous methyl tert-butyl ether, and the temperature was controlled at 15 to 25 °C. After the dropwise addition, the temperature was raised to room temperature and the stirring was continued for 5 hours until the raw materials were reacted completely. The reaction system was cooled in an ice bath to 10 °C, slowly added with 60 ml of methanol, and the stirring was continued for 30 minutes. The resultant was washed with 0.5 N hydrochloric acid (2 × 600 ml), and saturated saline (2 × 500 ml) in order, dried with anhydrous sodium sulfate, and then the solvent was removed under reduced pressure to obtain 112 g of a light yellow oil (compound 3), with a yield of 85% and a chiral ee value of 97.2%. ¹H NMR (400 MHz, CDCl₃) δ 7.97 (d, *J =* 8.3 Hz, 2H), 7.42 (d, *J =* 8.3 Hz, 2H), 5.02 - 4.91 (m, 1H), 1.88 (d, *J* = 3.4 Hz, 1H), 1.59 (s, 9H), 1.49 (d, *J* = 6.5 Hz, 3H); ESI-MS: m/z = 223[M+H]⁺.

tert-butyl (R)-4-(1-hydroxyethyl) benzoate (3, 111.5 g, 501 mmol), 6-chloropyridazinone (62.3 g, 477 mmol) and triphenylphosphine (PPh₃, 150.2 g, 572 mmol) as the reactants were placed in a 3L three necked flask. Under the protection of N₂, 600 mL of anhydrous THF was added to dissolve them, then the reaction system was cooled to 0 °C, and diisopropyl azodicarboxylate (DIAD, 115.8 g, 572 mmol) was slowly added dropwise, and reacted overnight at room temperature. The solid in the reaction solution was filtered and the filtrate was added with 200 ml of ethyl acetate and 1000 ml of n-heptane, and the resultant was stirred at room temperature for 1 hour and suction filtered. 200 ml of ethyl acetate and 1000 ml of n-heptane were added again to the filtrate, slurried and suction filtered. The filter cake was washed with a mixed solvent of ethyl acetate: n-heptane=5:1 (600 mL), and the solvent was removed under reduced pressure to obtain 178.9 g of a yellow oil (compound 4), which was directly used for the next reaction without purification. ESI-MS: m/z=335 [M+H]⁺.

The crude tert-butyl (S)-4-(1-(3-chloro-6-oxopyridazine-1(6H)-yl)ethyl) benzoate as obtained above (4, 177 g), (4-(dimethylamino)methyl)phenyl)boric acid hydrochloride (5, 88.9 g, 412 mmol), Pd(dppf)Cl₂ (12.0 g, 17 mmol) and potassium carbonate (104 g, 755 mmol) were added into 1,4-dioxane (1000 ml) and water (100 ml), and after the air was replaced with nitrogen, the mixture was heated to 85 °C and reacted for 6 h. The reaction solution was cooled to room temperature, and solid impurities were filtered, and the filtrate was concentrated under reduced pressure to dryness. Ethyl acetate (EtOAc, 1200 ml) and water (800 ml) were added thereto to separate the organic layer. Acetic acid (59 ml) was added and the resultant was stirred at room temperature for 30 minutes, and then water (800 ml) was added, and the aqueous phase was cooled to 4 °C in an ice bath, added dropwise with a 2N sodium hydroxide solution to adjust the pH to 10-11, and extracted with ethyl acetate (1200 ml). The organic layer was filtered through silica gel, and removed off the solvent under reduced pressure, to obtain 100 g of a crude product (compound 6), with a two-step yield of 48%. ¹H NMR (400 MHz, CDCl₃) δ 7.94 (d, *J =* 8.4 Hz, 2H), 7.72 (d, *J* = 8.3 Hz, 2H), 7.65 (d, *J =* 9.7 Hz, 1H), 7.52 (d, *J* = 8.4 Hz, 2H), 7.40 (d, *J=* 8.2 Hz, 2H), 6.98 (d, *J=* 9.7 Hz, 1H), 6.44 (q, *J=* 14.1, 6.9 Hz, 1H), 3.46 (s, 2H), 2.26 (s, 6H), 1.86 (d, *J =* 7.0 Hz, 3H), 1.57 (s, 9H); ESI-MS: m/z = 434 [M+H]⁺.

### Tert-butyl (S)-4-(1-(3-(4-((dimethylamino)methyl)phenyl)-6-oxopyridazine-1(6H)-yl)ethyl)

benzoate (6, 100 g, 230 mmol) was dissolved in 175 ml of CH₂Cl₂, cooled to 0 °C in an ice bath, added with trifluoroacetic acid (230 ml), and reacted at room temperature for 3 hours. The reaction solution was evaporated off the solvent under reduced pressure, and 500 ml of methyl tert-butyl ether was added for slurrying, and the supernatant was poured out, such procedures were repeated for three times, and finally the solvent was removed under reduced pressure to obtain 80 g of a white solid (compound 7), with a yield of 71%.¹H NMR (400 MHz, CDCl₃) δ 12.74 (s, 1H), 8.03 (d, *J=* 8.4 Hz, 2H), 7.83 (d, *J =* 8.3 Hz, 2H), 7.65 (d, *J =* 9.7 Hz, 1H), 7.54 (d, *J =* 8.3 Hz, 4H), 7.07 (d, *J =* 9.7 Hz, 1H), 6.47 (q, *J* = 7.0 Hz, 1H), 4.26 (d, *J=* 8.5 Hz, 2H), 2.83 (s, 6H), 1.87 (d, *J* = 7.1 Hz, 3H); ESI-MS: m/z = 378 [M+H]⁺.

(*S*)-4-(1-(3-(4-((dimethylamino)methyl)phenyl)-6-oxopyridazine-1(6*H*)-yl)ethyl)benzoi c acid trifluoroacetate (7, 0.79 g, 1.60 mmol) was placed in a 25 ml three necked flask, 2 ml of dimethylformamide (DMF) was added to dissolve, and 1-hydroxybenzotriazole (HOBT, 0.26 g, 1.93 mmol) and N-(3-dimethylaminopropyl)-N'-ethylcarbodiimide hydrochloride (EDCI, 0.46 g, 2.41 mmol) were added immediately, and stirred at room temperature for 30 minutes. A solution of 4-fluoro-1,2-phenylenediamine (0.24 g, 1.93 mmol) in 1.2 ml of DMF and diisopropylethylamine (0.79 ml, 4.82 mmol) were added dropwise under N₂ protection. The reaction system was stirred at room temperature overnight, and a 10% potassium carbonate solution (6.6 ml) and dichloromethane (15.0 ml) were added to the reaction solution to separate the organic phase. 3N hydrochloric acid (6.4 ml) was added and stirred at room temperature for 2 hours to separate the aqueous phase. The aqueous phase was cooled in an ice bath to 4 °C, added dropwise with a 2N sodium hydroxide solution to adjust the pH to 10-11, and extracted with dichloromethane (25 ml). The organic phase was removed off the solvent under reduced pressure to obtain a crude, which was separated with reverse phase column chromatography (C18 column, methanol-water system) to obtain 0.32g of a white solid (compound **I-1**), with a chiral ee value of 97% and a yield of 41%.¹H NMR (400 MHz, CDCl₃) δ 7.97 (s, 1H), 7.85 (d, *J* = 8.1 Hz, 2H), 7.73 (d, *J* = 8.2 Hz, 2H), 7.65 (d, *J=* 9.7 Hz, 1H), 7.56 (d, *J* = 8.0 Hz, 2H), 7.41 (d, *J =* 8.2 Hz, 2H), 7.13 (dd, *J =* 8.2, 6.1 Hz, 1H), 6.94 (d, *J* = 9.7 Hz, 1H), 6.49 (dt, *J =* 12.2, 5.5 Hz, 2H), 6.42 (dd, *J =* 14.1, 7.1 Hz, 1H), 4.00 (s, 2H), 3.46 (s, 2H), 2.26 (s, 6H), 1.87 (d, *J =* 7.0 Hz, 3H); ESI-MS: m/z = 486 [M+H]⁺.

### Examples 2-10: Synthesis of Compounds I-2 to I-10

Compounds I-2 to I-10 were prepared using a method similar to that in Example 1 as follows:

| Compound | Structure, preparation method, ¹H NMR and MS data |
|---|---|
| I-2 | 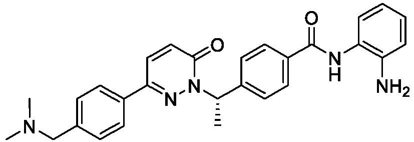 Compound **I-2** (white solid, yield 68%) was prepared by the same method as that in Example 1, except that 4-fluoro-1,2-phenylenediamine was replaced with o-phenylenediamine. |
| | ¹H NMR (400 MHz, CDCl₃) δ 7.94 (s, 1H), 7.86 (d, *J =* 8.1 Hz, 2H), 7.73 (d, *J* = 8.3 Hz, 2H), 7.66 (d, *J* = 9.7 Hz, 1H), 7.59 (d, *J =* 8.2 Hz, 2H), 7.41 (d, *J* = 8.2 Hz, 2H), 7.30 (d, *J =* 8.0 Hz, 1H), 7.13 - 7.04 (m, 1H), 6.98 (d, *J* = 9.7 Hz, 1H), 6.88 - 6.79 (m, 2H), 6.44 (q, *J =* 7.1 Hz, 1H), 3.85 (s, 2H), 3.47 (s, 2H), 2.26 (s, 6H), 1.88 (d, *J =* 7.1 Hz, 3H). ESI-MS: m/z = 468 [M+H]⁺. |
| I-3 | 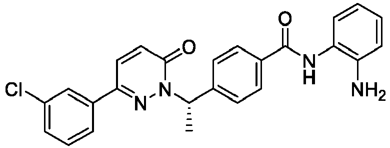 Compound I-3 (white solid, yield 56%) was prepared by the same method as that in Example 1, except that (4-((dimethylamino)methyl)phenyl)boric acid hydrochloride was replaced with 3-chlorophenylboronic acid, and 4-fluoro-1,2-phenylenediamine was replaced with 1,2-phenylenediamine. |
| | ¹H NMR (400 MHz, CDCl₃) δ 8.34 (s, 1H), 7.84 (d, *J* = 6.4 Hz, 2H), 7.75 (s, 1H), 7.67 - 7.61 (m, 1H), 7.59 (d, *J* = 9.6 Hz, 1H), 7.50 (d, *J* = 7.3 Hz, 2H), 7.42 - 7.36 (m, 2H), 7.24 (d, *J* = 7.1 Hz, 1H), 7.04 (t, *J* = 7.6 Hz, 1H), 6.92 (d, *J* = 9.6 Hz, 1H), 6.83 - 6.72 (m, 2H), 6.39 (q, *J* = 7.0 Hz, 1H), 3.90 (s, 2H), 1.84 (t, *J* = 7.1 Hz, 3H). ESI-MS: m/z = 445 [M+H]⁺. |
| I-4 | 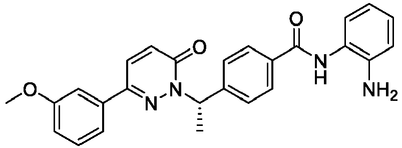 Compound I-4 (white solid, yield 49%) was prepared by the same method as that in Example 1, except that (4-((dimethylamino)methyl)phenyl)boric acid hydrochloride was replaced with 3-methoxyphenylboronic acid, and 4-fluoro-1,2-phenylenediamine was replaced with 1,2-phenylenediamine. |
| | ¹H NMR (400 MHz, CDCl₃) δ 8.62 (d, *J* = 8.5 Hz, 1H), 7.82 (d, *J* = 8.0 Hz, 2H), 7.58 (d, *J* = 9.3 Hz, 1H), 7.46 (d, *J* = 7.4 Hz, 2H), 7.36 (t, *J* = 8.1 Hz, 1H), 7.33 - 7.29 (m, 2H), 7.20 (d, *J* = 7.6 Hz, 1H), 7.00 (t, *J* = 7.6 Hz, 1H), 6.96 (dd, *J=* 8.0, 1.3 Hz, 1H), 6.86 (dd, *J =* 9.7, 1.3 Hz, 1H), 6.73 (t, *J* = 7.9 Hz, 2H), 6.36 (q, *J* = 7.0 Hz, 1H), 4.10 - 3.69 (br m, 5H), 1.82 (d, *J =* 7.0 Hz, 3H). ESI-MS: m/z = 441 [M+H]⁺. |
| I-5 | 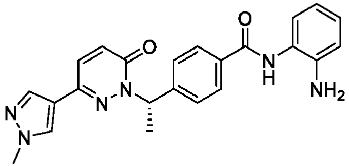 Compound I-5 (white solid, yield 42%) was prepared by the same method as that in Example 1, except that (4-((dimethylamino)methyl)phenyl)boric acid hydrochloride was replaced with 1-methyl-1*H*-pyrazole-4-boric acid, and 4-fluoro-1,2-phenylenediamine was replaced with 1,2-phenylenediamine. |
| | ¹H NMR (400 MHz, CDCl₃) δ 8.29 (s, 1H), 7.83 (d, *J* = 8.0 Hz, 2H), 7.78 (s, 1H), 7.72 (s, 1H), 7.48 (d, *J* = 7.6 Hz, 2H), 7.34 (d, *J* = 9.6 Hz, 1H), 7.26 (d, *J* = 7.6 Hz, 1H), 7.05 (t, *J* = 7.6 Hz, 1H), 6.86 (d, *J* = 9.6 Hz, 1H), 6.83 - 6.74 (m, 2H), 6.35 (q, *J* = 6.9 Hz, 1H), 3.93 (s, 3H), 1.80 (d, *J* = 7.0 Hz, 3H). ESI-MS: m/z = 415 [M+H]⁺. |
| I-6 | 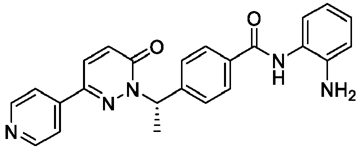 Compound I-6 (white solid, yield 45%) was prepared by the same method as that in Example 1, except that (4-((dimethylamino) methyl)phenyl)boric acid hydrochloride was replaced with 4-pyridine boronic acid pinacol ester, and 4-fluoro-1,2-phenylenediamine was replaced with 1,2-phenylenediamine. |
| | ¹H NMR (400 MHz, CDCl₃) δ 8.67 (d, *J* = 6.1 Hz, 2H), 8.52 (s, 1H), 7.84 (d, *J* = 8.1 Hz, 2H), 7.71 - 7.61 (m, 3H), 7.51 - 7.41 (m, 2H), 7.20 (d, *J* = 7.6 Hz, 1H), 7.04 - 6.98 (m, 1H), 6.95 (d, *J* = 9.7 Hz, 1H), 6.79 - 6.68 (m, 2H), 6.39 (q, *J* = 7.0 Hz, 1H), 3.89 (s, 2H), 1.85 (d, *J* = 7.1 Hz, 3H). ESI-MS: m/z = 412 [M+H]⁺. |
| I-7 | 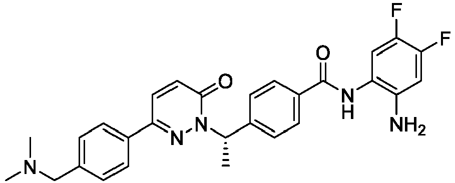 Compound I-7 (white solid, yield 52%) was prepared by the same method as that in Example 1, except that 4-fluoro-1,2-phenylenediamine was replaced with 4,5-difluoro-1,2-phenylenediamine. |
| | ¹H NMR (400 MHz, CDCl₃) δ 8.82 (s, 1H), 7.81 (d, *J* = 8.2 Hz, 2H), 7.71 (d, *J* = 8.2 Hz, 2H), 7.60 (d, *J* = 9.7 Hz, 1H), 7.44 (d, *J* = 8.2 Hz, 2H), 7.39 (d, *J* = 8.2 Hz, 2H), 7.11 (dd, *J* = 10.7, 8.5 Hz, 1H), 6.84 (d, *J* = 9.7 Hz, 1H), 6.50 (dd, *J* = 11.5, 7.6 Hz, 1H), 6.32 (q, *J* = 6.9 Hz, 1H), 3.90 (s, 2H), 3.46 (s, 2H), 2.24 (s, 6H), 1.82 (d, *J* = 7.0 Hz, 3H). ESI-MS: m/z = 504 [M+H]⁺. |
| I-8 | 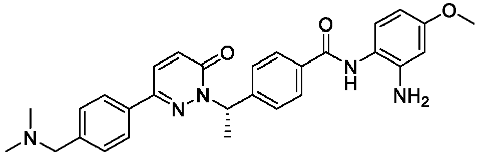 Compound I-8 (white solid, yield 52%) was prepared by the same method as that in Example 1, excent that 4-fluoro-1,2-phenylenediamine was replaced with 4-methoxy-1,2-phenylenediamine. |
| | ¹H NMR (400 MHz, DMSO-*d*₆) δ 9.46 (s, 1H), 8.06 (d, *J* = 9.7 Hz, 1H), 7.92 (d, *J* = 8.1 Hz, 2H), 7.88 (d, *J* = 8.0 Hz, 2H), 7.49 (d, *J* = 8.1 Hz, 2H), 7.41 (d, *J* = 8.0 Hz, 2H), 7.06 (d, *J* = 9.7 Hz, 1H), 6.98 (d, *J* = 8.6 Hz, 1H), 6.36 - 6.26 (m, 2H), 6.19 - 6.09 (m, 1H), 4.90 (s, 2H), 3.67 (s, 3H), 3.44 (s, 2H), 2.16 (s, 6H), 1.81 (d, *J =* 7.0 Hz, 3H). ESI-MS: m/z = 498 [M+H]⁺. |
| I-9 | 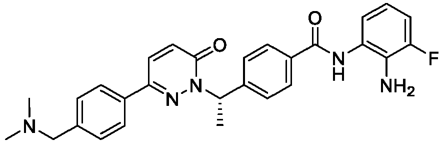 Compound I-9 (white solid, yield 48%) was prepared by the same method as that in Example 1, except that 4-fluoro-1,2-phenylenediamine was replaced with 3-fluoro-1,2-phenylenediamine. |
| | ¹H NMR (400 MHz, CDCl₃) δ 8.39 (s, 1H), 7.85 (d, *J* = 8.0 Hz, 2H), 7.72 (d, *J* = 8.2 Hz, 2H), 7.63 (d, *J* = 9.7 Hz, 1H), 7.53 (d, *J* = 8.2 Hz, 2H), 7.41 (d, *J* = 8.0 Hz, 2H), 7.08 (d, *J =* 8.1 Hz, 1H), 6.96 - 6.86 (m, 2H), 6.76 - 6.65 (m, 1H), 6.40 (q, *J* = 7.0 Hz, 1H), 3.97 (s, 2H), 3.47 (s, 2H), 2.26 (s, 6H), 1.86 (d, *J =* 7.0 Hz, 3H). ESI-MS: m/z = 486 [M+H]⁺. |
| I-10 | 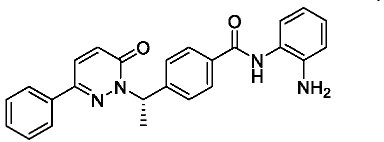 Compound 1-10 (white solid, yield 51%) was prepared by the same method as that in Example 1, except that (4-((dimethylamino)methyl)phenyl)boric acid hydrochloride was replaced with phenylboronic acid, and 4-fluoro-1,2-phenylenediamine was replaced with 1,2-phenylenediamine. |
| | ¹H NMR (400 MHz, DMSO) δ 9.62 (s, 1H), 8.08 (d, *J* = 9.8 Hz, 1H), 7.95 (dd, *J* = 7.8, 6.3 Hz, 4H), 7.57 - 7.44 (m, 5H), 7.16 (d, *J =* 7.3 Hz, 1H), 7.09 (d, *J* = 9.7 Hz, 1H), 7.01 - 6.93 (m, 1H), 6.77 (dd, *J* = 8.0, 1.3 Hz, 1H), 6.63 - 6.54 (m, 1H), 6.35 (q, *J =* 7.0 Hz, 1H), 4.90 (s, 2H), 1.83 (d, *J =* 7.0 Hz, 3H). ESI-MS: m/z = 411 [M+H]⁺. |

### Example 11: Synthesis of Compound I-11

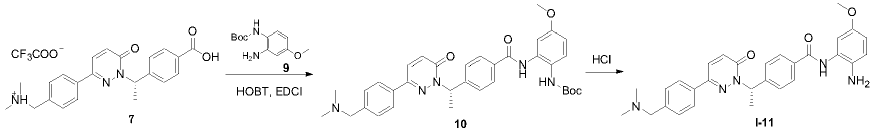

(*S*)-4-(1-(3-(4-((dimethylamino)methyl)phenyl)-6-oxopyridazine-1(6*H*)-yl)ethyl)benzoi c acid trifluoroacetate (7, 0.79 g, 1.60 mmol) was placed in a 25 ml three neck flask, 2 ml of DMF was added to dissolve, and 1-hydroxybenzotriazole (HOBT, 0.26 g, 1.93 mmol), N-(3-dimethylaminopropyl)-N'-ethylcarbodiimide hydrochloride (EDCI, 0.46 g, 2.41 mmol) were added immediately, and stirred at room temperature for 30 minutes. Under the protection of N₂, a solution of tert-butyl (2-amino-4-methoxy phenyl)carbamate (0.46 g, 1.93 mmol) in 1.2 ml of DMF and diisopropylethylamine (0.79 ml, 4.82 mmol) were added dropwise. The resultant was stirred at room temperature overnight, and a 10% potassium carbonate solution (6.6 ml) and dichloromethane (15.0 ml) were added to the reaction solution to separate the organic phase. 3N hydrochloric acid (6.4 ml) was added and stirred at room temperature for 2 hours to separate the aqueous phase, which was cooled in an ice bath to 4 °C, added dropwise with a 2N sodium hydroxide solution to adjust the pH to 10-11, and extracted with dichloromethane (25 ml). The organic phase was removed off the solvent under reduced pressure to obtain a crude, which was separated with column chromatography to obtain 0.68 g of a white solid (intermediate 10), with a yield of 73%. ¹H NMR (400 MHz, CDCl₃) δ 8.05 - 7.96 (m, 1H), 7.91 (d, *J* = 8.3 Hz, 2H), 7.72 (d, *J* = 8.3 Hz, 2H), 7.64 (d, *J* = 9.7 Hz, 1H), 7.55 (d, *J* = 8.3 Hz, 2H), 7.45 (s, 1H), 7.42 (d, *J* = 8.3 Hz, 2H), 7.08 (d, *J* = 8.8 Hz, 1H), 7.04 - 7.01 (m, 1H), 6.98 (d, *J =* 9.7 Hz, 1H), 6.86 (s, 1H), 6.44 (q, *J* = 7.0 Hz, 1H), 3.75 (s, 3H), 3.55 (s, 2H), 2.30 (s, 6H), 1.86 (d, *J* = 7.0 Hz, 3H), 1.48 (s, 9H); ESI-MS: m/z = 598 [M+H]⁺.

The intermediate **10** (0.68 g, 1.16 mmol) was dissolved in 2 ml of CH₂Cl₂, cooled to 0 °C in an ice bath, and a HCl saturated dioxane solution (10 ml) was added. The reaction was carried out at room temperature for 3 hours. The reaction solution was evaporated off the solvent under reduced pressure, and 20 ml of saturated sodium bicarbonate solution was added, followed by ethyl acetate extraction (25 ml × 2). The combined organic phase was washed with saturated saline (15 ml × 1), dried with anhydrous sodium sulfate and removed off the solvent under reduced pressure, and the resultant was separated by reverse phase column chromatography (C18 column, methanol-water system) to obtain 0.43 g of a white solid (compound **I-11**), with a yield of 75%.¹H NMR (400 MHz, CDCl₃) δ 8.35 (s, 1H), 7.86 (d, *J* = 8.2 Hz, 2H), 7.73 (d, *J* = 8.2 Hz, 2H), 7.65 (d, *J* = 9.7 Hz, 1H), 7.58 (d, *J* = 8.2 Hz, 2H), 7.41 (d, *J* = 8.2 Hz, 2H), 7.31 (d, *J* = 2.9 Hz, 1H), 6.97 (d, *J* = 9.7 Hz, 1H), 6.80 (d, *J* = 8.6 Hz, 1H), 6.63 (dd, *J* = 8.6, 2.9 Hz, 1H), 6.43 (q, *J=* 7.0 Hz, 1H), 3.75 (s, 3H), 3.47 (s, 2H), 3.38 (s, 2H), 2.26 (s, 6H), 1.88 (d, *J=* 7.1 Hz, 3H). ESI-MS: m/z = 498 [M+H]⁺.

### Example 12: Synthesis of Compound 1-12

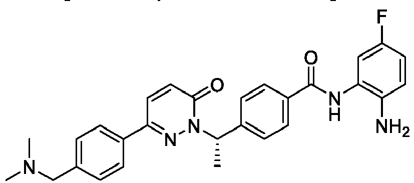

Compound I-12 (white solid, yield 72%) was prepared by the same method as that in Example 11, except that tert-butyl (2-amino-4-methoxyphenyl)carbamate was replaced with tert-butyl (2-amino-4-fluorophenyl)carbamate.

¹H NMR (400 MHz, CDCl₃) δ 8.24 (s, 1H), 7.85 (d, *J* = 8.3 Hz, 2H), 7.74 (d, *J* = 8.3 Hz, 2H), 7.66 (d, *J* = 9.7 Hz, 1H), 7.58 (d, *J* = 8.3 Hz, 2H), 7.48 - 7.37 (m, 3H), 6.98 (d, *J* = 9.7 Hz, 1H), 6.85 - 6.72 (m, 2H), 6.43 (q, *J* = 7.1 Hz, 1H), 3.49 (s, 2H), 2.28 (s, 6H), 1.89 - 1.86 (m, 5H); ESI-MS: m/z = 486 [M+H]⁺.

### Example 13: Synthesis of Compound I-13

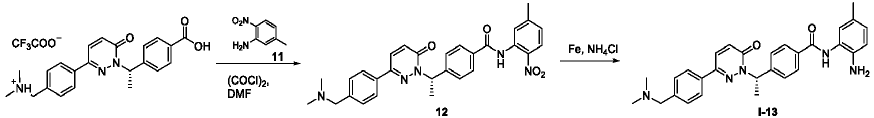

(*S*)-4-(1-(3-(4-((dimethylamino)methyl)phenyl)-6-oxopyridazine-1(6H)-yl)ethyl)benzoi c acid trifluoroacetate (7, 0.25 g, 0.51 mmol) was placed in a 25 ml three neck flask, 3 ml of anhydrous DCM was added under the protection of N₂, and cooled in an ice bath to 0 °C, and then a drop of anhydrous DMF was added and oxalyl chloride (52 µL. 0.61 mmol) was gradually added dropwise. The resultant was stirred at room temperature for 30 minutes, and then the solvent was evaporated off under reduced pressure. 8 ml of anhydrous DCM, 5-methyl-2-nitroaniline (0.093 g, 0.61 mmol), and diisopropylethylamine (252 µL, 1.53 mmol) were added. The reaction solution was stirred at room temperature overnight, saturated sodium bicarbonate solution (15 ml) and dichloromethane (15 ml) were added thereto to separate the organic phase, which was washed with saturated saline (15 ml), dried with anhydrous sodium sulfate and removed off the solvent under reduced pressure. The resultant crude was separated by column chromatography to obtain 0.21 g of a light yellow solid (intermediate **12**), with a yield of 80%.¹H NMR (400 MHz, CDCl₃) δ 11.39 (s, 1H), 8.80 (s, 1H), 8.15 (d, *J=* 8.6 Hz, 1H), 7.94 (d, *J* = 8.3 Hz, 2H), 7.74 (d, *J* = 8.2 Hz, 2H), 7.67 (d, *J* = 9.7 Hz, 1H), 7.63 (d, *J* = 8.3 Hz, 2H), 7.41 (d, *J* = 8.2 Hz, 2H), 7.05 - 6.96 (m, 2H), 6.45 (q, *J* = 7.0 Hz, 1H), 3.49 (s, 2H), 2.46 (s, 3H), 2.26 (s, 6H), 1.89 (d, *J* = 7.1 Hz, 3H); ESI-MS: m/z = 512 [M+H]⁺.

The intermediate **12** (0.20 g, 0.4 mmol) was dissolved in 2 ml of ethanol and 0.4 ml of water. Iron powder (0.11 g, 2.0 mmol) and ammonium chloride (0.21 g, 4.0 mmol) were added, heated and refluxed under N₂ protection for 5 hours. The reaction solution was cooled to room temperature, filtered with diatomite, and the filter cake was washed twice with ethanol. The filtrate was evaporated off the solvent under reduced pressure, added with saturated sodium bicarbonate solution (20 ml) and dichloromethane (30 ml). The organic phase was separated, washed with saturated saline (20 ml), dried with anhydrous sodium sulfate and removed off the solvent under reduced pressure. The resultant was separated by reverse phase column chromatography (C18 column, methanol-water system) to obtain 0.12 g of a white solid (compound 1-13), with a yield of 62%.¹H NMR (400 MHz, CDCl₃) δ 8.47 (s, 1H), 7.82 (d, *J* = 8.0 Hz, 2H), 7.72 (d, *J* = 8.2 Hz, 2H), 7.61 (d, *J* = 9.7 Hz, 1H), 7.49 (d, *J* = 7.6 Hz, 2H), 7.40 (d, *J* = 8.1 Hz, 2H), 7.08 (s, 1H), 6.89 (dd, *J* = 9.6, 2.3 Hz, 1H), 6.81 (d, *J* = 7.9 Hz, 1H), 6.66 (d, *J* = 7.9 Hz, 1H), 6.38 (q, *J* = 6.6 Hz, 1H), 3.67 (s, 2H), 3.45 (s, 2H), 2.24 (s, 6H), 2.17 (s, 3H), 1.84 (d, *J* = 6.9 Hz, 3H); ESI-MS: m/z = 482 [M+H]⁺.

### Pharmacological Experiment Examples

**HYH-073 (racemate of I-1), HYH-072 (racemate of 1-2), 1-3 (racemic), 1-4 (racemic), and I-10 (racemic)** were prepared according to the methods disclosed in CN109280032A.

**I-1 (R-configuration), I-2 (R-configuration), and I-10 (R-configuration)** were obtained from commercial companies through the resolution of **I-1 (racemic), I-2 (racemic), and I-10 (racemic)** prepared above.
**MS-275** and **Chidamide** were purchased from MCE MedChemExpress;

### Pharmacological Experiment Example 1:

*In vitro* assay of histone deacetylase activity

Human recombinant HDAC family proteins with His and GST tags were expressed using insect baculovirus expression system, and a biologically active HDAC family recombinant protein was obtained through Ni affinity column protein purification. HTRF detection method was used with H3(1-21) K9Ac biotin as the substrate. HDAC protein, gradient diluted compounds and the substrate were added to a white 384-well flat-bottomed microplate (ProxiPlate-384 Plus, PerkinElmer) and reacted for 1 hour, and a mixture of Eu labeled H3K9 antibody and XL665 labeled streptavidin were added and equilibrated for 0.5 hours at room temperature. By using the principle of HTRF, time-resolved fluorescence at 615nm and 665nm was detected using a microplate reader, and the ratio therebetween was calculated, and the corresponding enzyme activity inhibition rate was calculated by using GraphPad for analysis. Briefly, 20 µl of the activity testing system included the HDAC substrate (0.2 µM. 4 µl), the Human Recombinant Protein HDAC (2-5ng)/µl. 4 µl), the compound (2 µl), a mixture (10 µl) of Eu labeled H3K9 antibody and XL665 labeled streptavidin, and all components were diluted with Tris buffer (50 mM Tris-HCl, pH 8.0, 137mM NaCl, 2.7 mM KC1, and 1mM MgCl₂).

Pharmacological data: the pharmacological test results of the compounds of the present invention are shown in Table 1 below, and the controls used in the test were MS-275 and Chidamide.

**Table 1: Inhibitory activity of the compounds on HDAC1 and HDAC6**

| Compound No. | HDAC1 IC50(nM) | HDAC6 IC50(nM) | Compound No. | HDAC1 IC50(nM) | HDAC6 IC50(nM) |
|---|---|---|---|---|---|
| **I-1** | 57.57±4.42 | >10000 | **I-6** | 60.47±5.73 | >10000 |
| **HYH-073 (racemate of I-1)** | 80.54±16.74 | >10000 | **I-7** | 99.45±11.10 | >10000 |
| **I-1 (R configuration)** | 374.70±42.14 | >10000 | **I-8** | 126.05±18.31 | >10000 |
| **I-2** | 23.29±1.10 | >10000 | **I-9** | 122.35±11.10 | >10000 |
| **HYH-072 (racemate of I-2)** | 38.29±5.26 | >10000 | **I-10** | 50.45±13.68 | >10000 |
| **I-2(R configuration)** | 72.13±21.07 | >10000 | **I-10(R configuration)** | 154.55±5.73 | >10000 |
| **I-3** | 52.35±10.20 | >10000 | **I-11** | 42.42±0.80 | >10000 |
| **I-3(racemic)** | 81.93±8.43 | >10000 | **I-12** | 31.28±3.27 | >10000 |
| **I-4** | 35.21±4.47 | >10000 | **I-13** | 44.75±1.53 | >10000 |
| **I-4(racemic)** | 107.49+12.04 | >10000 | **MS-275** | 255.35+4.74 | >10000 |
| **I-5** | 124.15+24.40 | >10000 | **Chidamide** | 132.10+30.97 | >10000 |

From the above table, it can be seen that the molecular level test results of these S-configuration-containing compounds indicate that the compounds of the present invention have potent binding activity to HDAC1. Most of the compounds have inhibitory activity at molecular level better than that of the positive control MS-275, and better than or equivalent to that of the marketed drug Chidamide, and have higher selectivity for the HDAC6 subtype. In addition, the binding activity of the S-configuration compounds to HDAC1 is superior to that of R-configuration compounds, indicating that S-configuration compounds play a major role in the HDAC1 binding activity.

### Pharmacological Experiment Example 2: Antiproliferative Activity Test of Compounds Against Cancer Cellsin vitro

The tumor cells used were purchased from commercial cell banks (HCT116 purchased from ATCC, SKM 1 purchased from JCRB). The growth inhibition of cells was detected using the CCK-8 method. The specific steps were as follows. Cells in the logarithmic growth phase were seeded into a 96 well culture plate at an appropriate density, with 90 µL/well. After overnight cultivation, different concentrations of drugs were added for treatment for 72h, with each concentration in triplicate, and corresponding concentration of vehicle control and cell-free blank wells were also set. After the treatment, 10 µL of CCK-8 was added to each well and the plate was incubated in an incubator for 2-4 hours, and the optical density (OD value) at a wavelength of 450nm was measured using a SpectraMax 190 microplate reader. The inhibition rate of the drug on tumor cell proliferation was calculated according to the following equation: inhibition rate (%) = (OD_{control well} - OD_{treatment well})/OD_{control well} × 100%, and based on this, the IC₅₀ value was fitted using the 4-parameters method. The experiment was repeated twice, and the mean value and SD were calculated.

The experimental results are shown in Table 2 below (N. T. means untested).

With reference to HCT-116 cell proliferation inhibition test method, the inhibitory activity of the compounds on the proliferation of human myelodysplastic syndrome cell SKM-1 was tested. The experimental results are shown in Table 2 and Fig. 1. Fig. 1 shows the pharmacodynamic results of compound I-1 on the human myelodysplastic syndrome SKM-1 mice transplanted tumor model at different doses.

**Table 2: Antiproliferative Activity of the Compounds towards HCT-116 and SKM-1 Cell Lines**

| Compound No. | HCT-116 IC₅₀(µM) | SKM-1 IC₅₀(µM) | Compound No. | HCT-116 IC₅₀(µM) | SKM-1 IC₅₀(µM) |
|---|---|---|---|---|---|
| **I-1** | 0.22±0.004 | 0.14±0.008 | **I-6** | 0.33±0.004 | 0.24±0.011 |
| **HYH-073(racemate of** | 0.38±0.001 | 0.23±0.01 | **I-7** | 0.31±0.018 | 0.21±0.005 |
| **I-1 (R configuration)** | 1.55±0.094 | 1.06±0.079 | **I-8** | 0.87±0.037 | 0.49±0.016 |
| **I-2** | 0.08±0.002 | 0.06±0.001 | **I-9** | 0.50±0.065 | 0.34±0.013 |
| **HYH-072(racemate of** | 0.11±0.007 | 0.09±0.004 | **I-10** | 0.40±0.072 | NT |
| **I-2(R configuration)** | 0.30±0.001 | 0.29±0.006 | **I-10(R configuration)** | 1.16±0.072 | NT |
| **I-3** | 0.39±0.006 | 0.27±0.013 | **I-11** | 0.31±0.037 | 0.27±0.019 |
| **I-3(racemic)** | 0.65±0.032 | 0.49±0.005 | **I-12** | 0.14±0.004 | 0.10±0.000 |
| **I-4** | 0.23±0.004 | 0.16±0.001 | **I-13** | 0.22±0.024 | 0.17±0.002 |
| **I-4(racemic)** | 0.34±0.012 | 0.24±0.009 | **MS-275** | 1.48±0.008 | 0.39±0.000 |
| **I-5** | 0.28±0.008 | 0.20±0.001 | **Chidamide** | 1.70±0.105 | 0.55±0.004 |

| | | | | | |
|---|---|---|---|---|---|
| NT=not tested | | | | | |

From experimental results in Table 2, it can be seen that this class of compounds exhibited potent *in vitro* antiproliferative activity towards different types of cancer cell lines, superior to MS-275 and Chidamide. In addition, In comparison of I-1 and I-1 (R-configuration), I-2 and I-2 (R-configuration), and I-10 and I-10 (R-configuration), S-configuration compounds show significantly superior antiproliferative activity than that of R-configuration compounds, indicating that S-configuration plays a major role in cellular activity and these compounds can be developed as a novel class of anti-tumor drugs.

### Pharmacological Experiment Example 3: Multiple Subtypes of HDACs inhibitory activities of representative compounds

The testing method for biological activity was the same as the method mentioned in the Pharmacological Experiment Example 1, and the results are shown in Table 3 below.

**Table 3: Inhibitory Activities of some Compounds on HDAC2, HDAC3, HDAC8, and HDAC 10**

| Compound No. | IC₅₀ (µM) | | | |
|---|---|---|---|---|
| | HDAC2 | HDAC3 | HDAC8 | HDAC10 |
| **I-1** | 0.09±0.006 | 0.42±0.105 | >100 | >100 |
| **I-1(R configuration)** | 0.32±0.048 | 0.72±0.196 | >100 | >100 |
| **I-2** | 0.09±0.002 | 0.39±0.028 | >100 | >100 |
| **I-2(R configuration)** | 0.30±0.057 | 0.84±0.047 | >100 | >100 |
| **MS-275** | 0.75±0.042 | 1.58±0.025 | >100 | >100 |

From Table 3 above, S-configuration-containing compounds show potent inhibitory activity to HDAC2 and HDAC3, which is superior to the positive control MS-275; and weak inhibitory activity against HD AC 10 subtype, with a selectivity of greater than 100 times, indicating that these compounds are a class of highly selective class I HDAC inhibitors. In addition, the inhibitory activity of S-configuration compounds to HDAC2 and HDAC3 is superior to that of R-configuration compounds, indicating that S-configuration compounds play a major role in the binding activity to HDAC2 and HDAC3.

### Pharmacological Experiment Example 4: In vitro Antiproliferative Activity of Compound I-1 Against Various Cancer Cells

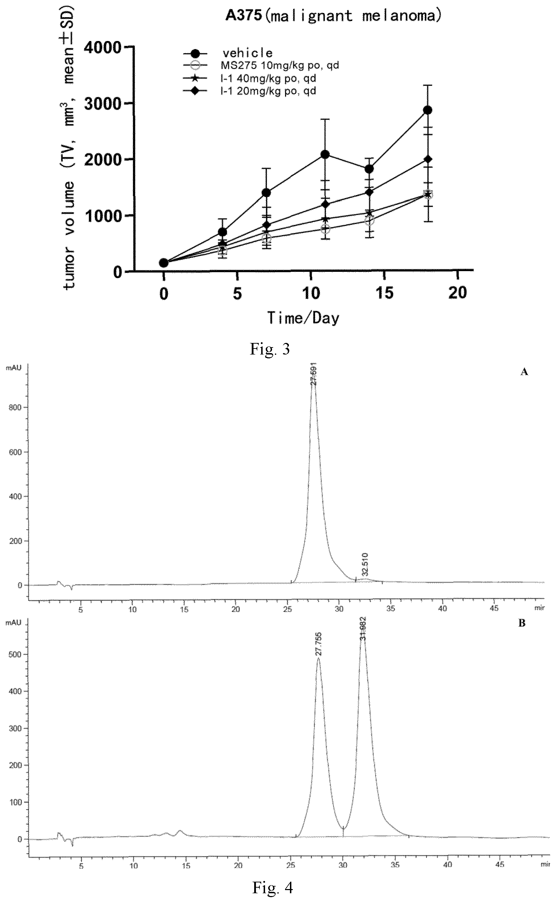

The testing method for antiproliferative activity was the same as that mentioned in Pharmacological Experiment Example 2, and the results are shown in Table 4 and Figs. 2-3. Fig. 2 shows the pharmacodynamic results of compound I-1 on human acute myeloid leukemia OCI-AML-3 mice transplanted tumor model. Fig. 3 shows the pharmacodynamic results of compound I-1 on human malignant Melanoma A375 mice transplanted tumor model.

**Table 4 Antiproliferative activity of Compound I-1 Against Various Cancer Cell Lines**

| Cell lines | Disease type | Cytotoxicity (IC₅₀ µM) |
|---|---|---|
| MV-4-11 | Acute monocyte/Monocyte leukemia | 0.088±0.002 |
| WILL-1 | Diffuse large B-cell lymphoma | 0.116±0.006 |
| OCI-AML3 | Human acute myeloid leukemia | 0.121±0.003 |
| CMK | Down syndrome acute megakaryocyte leukemia | 0.131±0.011 |
| Mino | Mantle cell lymphoma | 0.142±0.013 |
| EoL-1 | Chronic eosinophilic leukemia | 0.158 |
| OCI-LY-3 | Diffuse large B-cell lymphoma | 0.198±0.048 |
| U-2932 | Diffuse large B-cell lymphoma | 0.214±0.001 |
| HH | Primary cutaneous T-cell non Hodgkin lymphoma | 0.219±0.093 |
| RL | Diffuse large B-cell lymphoma | 0.24±0.019 |
| NU-DHL-1 | Diffuse large B-cell lymphoma | 0.243 |
| OCI-LY-10 | Diffuse large B-cell lymphoma | 0.297±0.042 |
| WILL-2 | Diffuse large B-cell lymphoma | 0.347±0.217 |
| Z138 | Human mantle cell lymphoma | 0.363±0.046 |
| REC-1 | Human mantle cell lymphoma | 0.381 |
| CCRF-CEM | T-cell acute lymphoblastic leukemia | 0.382±0.067 |
| RS4;11 | Acute lymphoblastic leukemia | 0.412±0.129 |
| MO-91 | Acute myeloid leukemia | 0.427±0.163 |
| U937 | Human tissue cell lymphoma | 0.435±0.254 |
| OPM-2 | Human multiple myeloid leukemia | 0.436±0.222 |
| SU-DHL-8 | Diffuse large B-cell lymphoma | 0.443±0.185 |
| K-562 | Chronic myeloid leukemia | 0.473±0.171 |
| KARPAS-1106P | Primary mediastinal large B-cell lymphoma | 0.498±0.009 |
| NOMO-1 | Acute monocytic leukemia | 0.523±0.12 |
| SU-DHL-6 | Diffuse large B-cell lymphoma | 0.576±0.066 |
| MOLP-8 | Human multiple myeloma | 0.632±0.066 |
| NCI-H929 | Human multiple myeloma | 0.642±0.025 |
| HDLM-2 | Hodgkin's lymphoma | 0.647±0.404 |
| H9 | Human T lymphocyte line | 0.649±0.044 |
| KMS-11 | Human multiple myeloma | 0.671±0.01 |
| HuT78 | Human T-lymphocytic leukemia | 0.769±0.461 |
| TMD8 | Diffuse large B-cell lymphoma | 0.807±0.142 |
| KG-1 | Acute myeloid leukemia | 0.95±0.066 |
| PC-9 | Lung cancer | 0.461±0.034 |
| CAKI-1 | Renal cell carcinoma | 0.594±0.011 |
| SNU-16 | gastric cancer | 0.675±0.064 |
| SNU-5 | Gastric cancer | 0.314±0.011 |
| SK-BR-3 | Breast cancer | 0.720±0.032 |
| MDA-MB-453 | Breast cancer | 0.498±0.036 |
| A375 | Melanoma | 0.556±0.018 |
| A2058 | Melanoma | 0.562±0.011 |
| VMM39 | Melanoma | 0.460±0.032 |
| VMM5A | Melanoma | 0.856±0.018 |
| SK-MEL-2 | Melanoma | 0.799±0.042 |

The results show that compound I-1 has potent and broad-spectrum antiproliferative activities against various blood cancer cell lines and solid tumor cell lines.

All references mentioned in the present invention are incorporated herein by reference in this application to the extent as if each reference is individually incorporated by reference. In addition, it should be understood that after reading the above disclosure of the present invention, those skilled in the art can make various modifications or amendments to the present invention, and such equivalents also fall within the scope of the claims attached to this application.

## Claims

1. A compound of formula I or a pharmaceutically acceptable salt thereof, 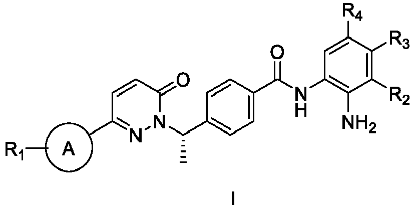 wherein
ring A is selected from a C₆-C₁₀ aryl group or a 5-10 membered heteroaryl group containing 1-3 heteroatoms selected from N, O, and S;
R₁ represents one or more substituents, each independently selected from H, halogen, C₁-C₆ linear or branched alkyl, C₁-C₆ linear or branched alkoxy, (Rₐ)(R_{b})N(CH₂)-; wherein Rₐ and R_{b} are each independently a C₁-C₃ alkyl, or form a 3-7-membered heterocyclyl together with the N atom connected therewith;
R₂, R₃, and R₄ are same or different from each other and are independently selected from hydrogen, halogen, C₁-C₃ alkyl, and C₁-C₃ alkoxy.

2. The compound or a pharmaceutically acceptable salt thereof according to claim 1, wherein, ring A is selected from phenyl, pyridyl, and pyrazolyl; R₁ is selected from 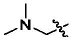 , halogen, C₁-C₃ alkoxy, and C₁-C₃ alkyl.

3. The compound or a pharmaceutically acceptable salt thereof according to claim 1 or 2, wherein, the compound is selected from the compounds of formula II, formula III and formula IV below: 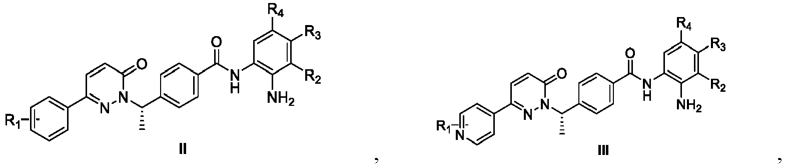 and 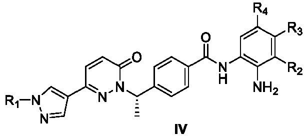 wherein, R₁, R₂, R₃, and R₄ are each defined as those in the corresponding claims.

4. The compound or a pharmaceutically acceptable salt thereof according to claim 1, wherein, the compound is selected from the following compounds:

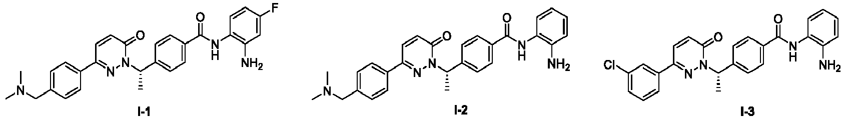

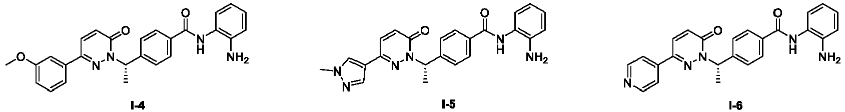

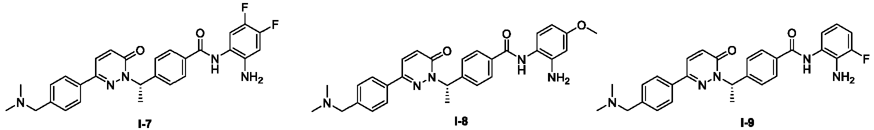

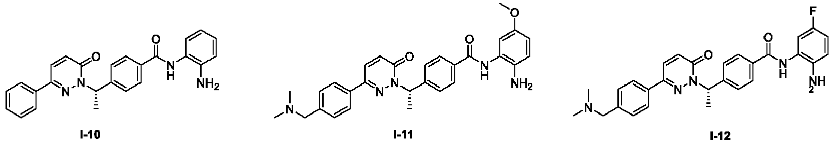

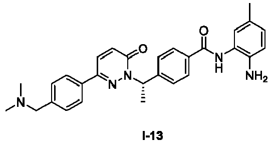

5. A method for preparing the compound of any one of claims 1 to 4, which is one of the following schemes, 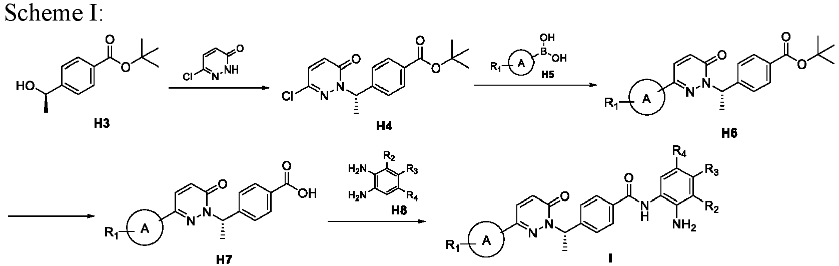 the method comprises steps of:
(1) reacting tert-butyl (R)-4-(1-hydroxyethyl)benzoate of **H3** with 6-chloropyridazinone via Mitsunobu reaction to obtain tert-butyl (*S*)-4-(1-(3-chloro-6-oxo-pyridazin-1(6*H*)-yl)ethyl)benzoate of **H4** with reverse configuration;
(2) reacting tert-butyl (*S*)-4-(1-(3-chloro-6-oxo-pyridazin-1(6*H*)-yl)ethyl)benzoate of **H4** with the compound of **H5** via Suzuki coupling reaction to obtain the compound of **H6**;
(3) removing the tert-butyl protection group of the compound of **H6** to obtain the compound of **H7**;
(4) reacting the compound **of H7** with the compound **of H8** via condensation reaction to obtain the compound of **formula I**; 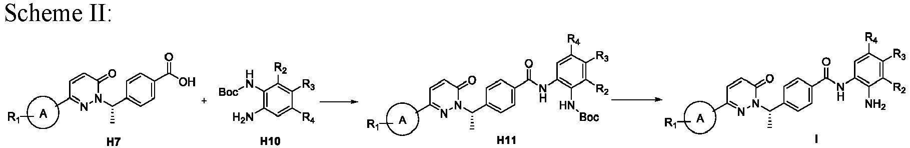
the method comprises steps of:
(1) reacting the compound of **H7** with the compound of **H10** via condensation reaction to obtain the compound of **H11**;
(2) removing the Boc protection group of the compound of **H11** to obtain the compound of **formula I**; 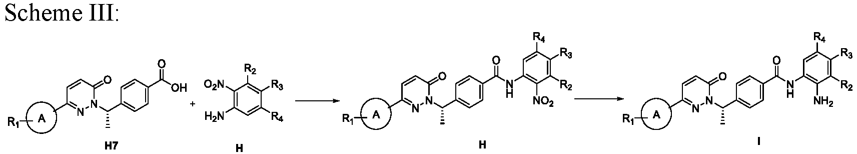
the method comprises steps of:
(1) reacting the compound of **H7** with the compound of **H13** via condensation reaction to obtain the compound of **H14;**
(2) reducing the compound of **H14** to obtain the compound of **formula I**;
wherein, ring A, R₁, R₂, R₃, and R₄ are each defined as those in the corresponding claims.

6. The method according to claim 5, wherein, **H3** is synthesized as follows: 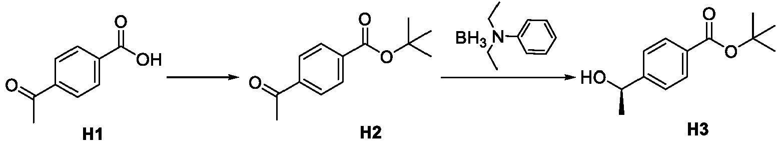
(1) p-acetylbenzoic acid of **H1** is esterified with Boc anhydride to obtain tert-butyl p-acetyl benzoate of **H2**;
(2) tert-butyl (R)-4-(1-hydroxyethyl) benzoate of **H3** is obtained from the reduction reaction of tert-butyl p-acetylbenzoate of **H2** with *N,N-*diethylaniline borane complex in the presence of (*S*)-2-methyl-CBS-oxazole borane ((*S*)-2-Me-CBS) as a chiral catalyst.

7. A pharmaceutical composition comprising at least one selected from the compound and the pharmaceutically acceptable salt thereof of any one of claims 1 to 4, and optionally a pharmaceutically acceptable carrier.

8. Use of the compound or the pharmaceutically acceptable salt thereof of any one of claims 1 to 4, or the pharmaceutical composition of claim 7 in preparation of a medicament for prevention or treatment of a disease associated with abnormal activity and expression of class I histone deacetylase.

9. Use according to claim 8, wherein, the disease associated with the abnormal activity and expression of class I histone deacetylase (class I HDAC) includes cancer, inflammation, neurodegenerative diseases, malaria and diabetes.

10. Use according to claim 9, wherein, the cancer is selected from the group consisting of myelodysplastic syndrome, leukemia, lymphomas, multiple myeloma, lung cancer, kidney cancer, gastric cancer, breast cancer, melanoma, colon cancer, liver cancer, ovarian cancer, pancreatic cancer, and rectal cancer,
in particular, the leukemia includes monocyte leukemia, acute myeloid leukemia, Down's syndrome acute megakaryocyte leukemia, T-cell acute lymphoblastic leukemia, acute lymphoblastic leukemia, multiple myeloid leukemia, chronic myeloid leukemia, human T-lymphocyte leukemia, and acute myeloid leukemia;
the lymphomas includes diffuse large B-cell lymphoma, mantle cell lymphoma, primary cutaneous T-cell non Hodgkin lymphoma, primary mediastinal large B-cell lymphoma, Hodgkin's lymphoma.
